# FASCICULE DE BREVET EUROPEEN

(11) **EP 1 409 165 B1**
(45) Date de publication et mention de la délivrance du brevet: **14.02.2007**
(21) Numéro de dépôt: 01947516.9
(22) Date de dépôt: 18.06.2001
(51) Int. Cl.: B09B 1/00, B01D 53/48, B01D 53/62, B01D 53/72, B01D 53/02

(54) **PRODUIT A BASE DE MACHEFERS D'INCINERATION DE DECHETS MENAGERS ET SON UTILISATION EN DECHARGE ET POUR L'EPURATION DU BIOGAZ**
PRODUKT AUF BASIS VON SCHLACKEN AUS EINER MÜLLVERBRENNUNGSANLAGE UND DESSEN VERWENDUNG IN EINER DEPONIE SOWIE REININGUNG DES BIOGASES
PRODUCT BASED ON CLINKERS FROM HOUSEHOLD REFUSE INCINERATION AND USE THEREOF IN LANDFILLS FOR PURIFYING BIOGAS

(30) Priorité: 19.06.2000 FR 0007814
(43) Date de publication de la demande: 21.04.2004
(73) Titulaire: NOVERGIE, 92000 Nanterre (FR)
(72) Inventeur: LUBETZKI, Jacques, F-92130 Issy les Moulineaux (FR); TROESCH, Olivier, 69680 Chassieu (FR); DE FREITAS, José, F-78420 Carrières sur Seine (FR)
(74) Mandataire: Michelet, Alain
(86) Numéro de dépôt international: PCT/FR2001/001899
(87) Numéro de publication internationale: WO 2001/097987

(56) Documents cités:
- WO-A-94/12444
- DE-A- 3 940 396
- DE-A- 19 648 368
- DE-C- 3 900 328
- US-A- 5 286 430
- US-A- 5 855 664
- PATENT ABSTRACTS OF JAPAN vol. 007, no. 112 (M-215), 17 mai 1983 (1983-05-17) & JP 58 033618 A (TSUNETAKA YASUKAGAWA;OTHERS: 01), 26 février 1983 (1983-02-26)
- BOGNER JEAN E: "UNDERSTANDING NATURAL AND INDUCED GAS MIGRATION THROUGH LANDFILL COVER MATERIALS - THE BASIS FOR IMPROVED LANDFILL GAS RECOVERY" 21ST INTERSOCIETY ENERGY CONVERSION ENGINEERING CONFERENCE: ADVANCING TOWARD TECHNOLOGY BREAKOUT IN ENERGY CONVERSION.;SAN DIEGO, CA, USA, 1986, pages 199-204, XP000955490 Proceedings of the Intersociety Energy Conversion Engineering Conference 21st. 1986 ACS, Washington, DC, USA

## Description

La présente invention concerne, d'une manière générale, un produit à base de mâchefer d'incinération de déchets ménagers et assimilés qui permet l'épuration du biogaz engendré par une décharge de déchets et qui est particulièrement utile pour former une couche terminale d'une décharge de déchets avant revégétalisation de celle-ci.

L'utilisation d'un tel produit pour l'épuration du biogaz est connue du document WO-A-94 12444.

Les mâchefers issus d'incinération des ordures ménagères sont des scories solides résultant de l'incinération de ces ordures. Ils se présentent sous la forme de granulés de couleur grise à noire.

Les mâchefers sont classés en fonction de leur potentiel de rejet. Depuis 1994, les mâchefers sont classés, en fonction du test de la norme NF X31-210, en trois catégories :
"V" : valorisable directement ;
"M" : valorisable après maturation ;
"F" : stockage en centre d'enfouissement de classe II.

Quoi qu'il en soit, les trois catégories sont définies sur la base des limites consignées dans le Tableau 1 ci-dessous.

**Tableau 1 : Catégories de classement des mâchefers**

| Paramètre | Catégorie V | Catégorie M | Catégorie S |
|---|---|---|---|
| Taux d'imbrûlés | <5 | <5 | >5 |
| Fraction soluble | <5 | 5 -10 | >10 |
| Mercure | <0.2 | 0.2 -0.4 | >0.4 |
| Plomb | <10 | 10 - 50 | >50 |
| Cadmium | <1 | 1 -2 | >2 |
| Arsenic | <2 | 2 -4 | >4 |
| Chrome hexavalent | <1.5 | 1.5 -3 | >3 |
| Sulfates | <10 000 | 10 000 -15 000 | >15 000 |
| COT | <1500 | 1500 - 2000 | >2000 |

| | | | |
|---|---|---|---|
| * rapporté au poids sec et exprimé en mg / kg (% pour le taux d'imbrûlés et la fraction soluble). | | | |

Jusqu'à ce jour, les mâchefers d'incinération d'ordures ménagères étaient soit simplement mis dans des décharges publiques ou privées, également appelées centres d'enfouissement techniques, sans préparation préalable, ou étaient traités et utilisés en sous-couche routière.

A l'heure actuelle, une quantité encore très importante des déchets sont simplement mis dans des décharges ou centres d'enfouissement techniques.

Le processus de dégradation anaérobie des matières organiques contenues dans les déchets stockés dans les décharges produit un mélange gazeux appelé "biogaz".

Ce biogaz est principalement composé de méthane, de gaz carbonique et d'autres gaz à l'état de traces, en particulier d'hydrogène sulfureux (H₂S).

Quelle que soit son origine, le biogaz non valorisé contribue, du fait de ses fortes teneurs en méthane, à l'effet de serre.

Le biogaz est considéré comme une source de nuisance en raison :
- des odeurs nauséabondes qui accompagnent son dégagement. En effet, le biogaz véhicule le plus souvent à l'état de traces (ppm) des composés soufrés (hydrogène sulfuré, polysulfure, etc.), des acides, des aldéhydes, responsables des odeurs les plus désagréables, ainsi que parfois du xylène, du toluène, du chlorure de vinyle, etc.
- de ses effets néfastes sur la végétation lors des opérations de reverdissement des sites remblayés (asphyxie des racines) ;
- des risques d'explosion (5 à 15 % de méthane dans l'air constituent un mélange explosif) ;
- de son impact sur l'effet de serre.

De ce fait, la gestion et la réhabilitation des décharges de déchets a nécessité l'emploi de systèmes de captation et de brûlage des biogaz. Mais ces systèmes de captation et de brûlage des biogaz nécessitent une surveillance pendant trente ans. En outre, certains des sites de décharge peuvent être utilisés pour la construction de bâtiments tels qu'usine d'incinération ou autre ensemble industriel, et les tassements de terrain inhérents à la construction peuvent conduire à la destruction des systèmes de captation du biogaz, rendant ainsi le site dangereux car pouvant localement engendrer des poches de gaz explosives.

Il serait donc souhaitable de disposer d'un produit convenant pour la réhabilitation de décharges qui pourrait être utilisé en complément, voire en remplacement des techniques de réhabilitation connues nécessitant des systèmes sensibles.

Il serait également souhaitable de disposer d'un produit d'épuration du biogaz permettant une récupération de méthane épuré, en particulier débarrassé des principaux gaz corrosifs généralement contenus dans le biogaz. Ainsi, on valoriserait le méthane récupéré tant du point de vue énergétique que d'une diminution de la corrosion des installations de valorisation.

La présente invention a donc pour objet un produit à base de mâchefers d'incinération de déchets ménagers et assimilés convenant pour la réhabilitation de décharges de déchets qui ne nécessite pas de système sensible de captation et brûlage du biogaz.

La présente invention a également pour objet une couche terminale, avant revégétalisation, d'une décharge de déchets constituée par le produit à base de mâchefers d'incinération de déchets ménagers et assimilés selon l'invention.

L'invention a encore pour objet un procédé d'épuration du biogaz engendré par une décharge de déchets qui consiste à faire passer le biogaz à travers une couche du produit à base de mâchefers selon l'invention.

La présente invention a aussi pour objet un procédé d'épuration du biogaz tel qu'indiqué ci-dessus pour l'obtention de méthane épuré à partir de ce biogaz et, en particulier débarrassé des composants odorants et corrosifs tels que les gaz chlorés et/ou soufrés.

Le produit à base de mâchefers d'incinération de déchets ménagers et assimilés, selon l'invention, se caractérise en ce qu'il comprend, en poids :
- 95 à 99,5 %, de préférence 98 à 99 % de mâchefers ayant une taille de particules égale ou inférieure à 40 mm et un taux d'humidité de 10 à 30 %, de préférence 15 à 20 % en poids ; et
- 0,5 à 5 %, de préférence 1 à 2 % de coke.

De préférence, les mâchefers d'incinération de déchets ménagers et assimilés convenant pour le produit de l'invention sont des mâchefers de classe V ou M selon la norme NF X31-210.

Le coke utile pour le produit selon l'invention peut être tout coke et plus particulièrement un coke de lignite ou un coke de thermolyse. Le produit à base de mâchefers selon l'invention peut être avantageusement utilisé comme couche intermédiaire ou terminale dans une décharge de déchets. De préférence, on utilise le produit à base de mâchefers selon l'invention comme couche terminale, avant revégétalisation, d'une décharge.

De préférence encore, la couche de produit à base de mâchefers selon l'invention est constituée de strates successives, légèrement compactées, de 30 à 40 cm d'épaisseur.

Ainsi, pour une hauteur de déchets de 5 mètres, on utilisera, de préférence, une couche constituée de une à trois strates du produit selon l'invention, d'une hauteur de 40 cm à 1 m environ.

Les produits à base de mâchefer selon l'invention peuvent être préparés de la manière suivante.

Les mâchefers issus de la combustion tombent à la sortie de la grille du four dans un extracteur rempli d'eau. Les mâchefers en ressortent humides. Cette humidité est généralement comprise entre 20 et 30 % en poids. Le mâchefer est, de préférence, ensuite déposé soit dans une fosse, soit dans une aire. Au cours de cette phase de stockage, il perd une partie de son humidité et on le récupère avant traitement complémentaire lorsqu'il a une humidité, de préférence comprise entre 15 et 20 % en poids.

Le mâchefer est alors criblé sur une grille à mailles de 40 mm et on ne récupère que le mâchefer le plus fin, c'est-à-dire de taille inférieure ou égale à 40 mm.

A ce mâchefer criblé, on rajoute la quantité voulue de coke pour obtenir dans le produit final 0,5 à 5 % en poids de coke. La quantité de coke ajoutée dépend de l'utilisation envisagée, couche intermédiaire, couche finale, substitution ou complément à d'autres systèmes de réhabilitation.

Le produit à base de mâchefer selon l'invention absorbe rapidement pratiquement la totalité du gaz carbonique et des composés odorants et/ou corrosifs, notamment les composés gazeux chlorés et/ou sulfurés, contenus dans le biogaz. L'abrasion des composants odorants, en particulier des gaz sulfurés, est mise en évidence par la disparition de l'odeur nauséabonde du biogaz.

L'élimination des composés odorants et/ou corrosifs du biogaz permet d'obtenir un méthane épuré qui peut être récupéré et valorisé pour la production d'énergie avec un risque moindre de corrosion des installations de récupération et de pollution de l'environnement.

## Revendications

1. Produit à base de mâchefer d'incinération de déchets ménagers et assimilés, **caractérisé en ce qu'**il comprend ; en poids :
- 95 à 99,5 % de mâchefers ayant une taille de particules égale ou inférieure à 40 mm, et un taux d'humidité de 10 à 30 %, de préférence de 15 à 20 % en poids ; et
- 0,5 à 5 % de coke.

2. Produit selon la revendication 1, **caractérisé en ce qu'**il comprend :
- 98 à 99 % de mâchefers ; et
- 1 à 2 % de coke.

3. Produit selon la revendication 1 ou 2, **caractérisé en ce que** le coke est un coke de lignite ou de thermolyse.

4. Produit selon l'une quelconque des revendications 1 à 3, **caractérisé en ce que** le mâchefer est un mâchefer de catégorie V ou M selon la norme NF X31-210.

5. Couche terminale, avant revégétalisation, d'une décharge de déchets, **caractérisée en ce qu'**elle est constituée du produit selon l'une quelconque des revendications 1 à 4.

6. Couche selon la revendication 5, **caractérisée en ce qu'**elle est constituée de strates successives, compactées, de 30 à 40 cm d'épaisseur.

7. Couche selon la revendication 6, **caractérisée en ce que**, pour une hauteur de déchets de 5 mètres, elle comprend une à trois strates pour une hauteur de 40 cm à 1 m.

8. Procédé d'épuration du biogaz engendré par une décharge de déchets, **caractérisé en ce qu'**il consiste à faire passer le biogaz à travers une couche du produit selon l'une quelconque des revendications 1 à 4.

9. Procédé selon la revendication 8, **caractérisé en ce que** la couche est constituée de strates successives, compactées, de 30 à 40 cm d'épaisseur.

10. Procédé d'épuration selon la revendication 9, **caractérisé en ce que**, pour une hauteur de déchets de 5 mètres, la couche comprend une à trois strates, du produit d'une hauteur de 40 cm à 1 m environ.

11. Procédé selon l'une quelconque des revendications 8 à 10, **caractérisé en ce que** la couche est une couche terminale, avant revégétalisation, de la décharge de déchets.

## Claims

1. Product based on scoria from the incineration of household and similar waste, **characterized in that** it comprises, by weight:
- 95 to 99.5% of scoria having a particle size equal to or less than 40 mm and a moisture level of 10 to 30%, preferably of 15 to 20%, by weight and
- 0.5 to 5% of coke.

2. Product according to Claim 1, **characterized in that** it comprises:
- 98 to 99% of scoria; and
- 1 to 2% of coke.

3. Product according to Claim 1 or 2, **characterized in that** the coke is a lignite coke or a thermolysis coke.

4. Product according to any one of Claims 1 to 3, **characterized in that** the scoria is a scoria from category V or M according to the NF X31-210 standard.

5. Final layer, before revegetation, of a waste landfill site, **characterized in that** it is composed of the product according to any one of Claims 1 to 4.

6. Layer according to Claim 5, **characterized in that** it is composed of successive and compacted strata with a thickness of 30 to 40 cm.

7. Layer according to Claim 6, **characterized in that**, for a waste height of 5 metres, it comprises one to three strata, for a height of 40 cm to 1 m.

8. Process for the purification of biogas generated by a waste landfill site, **characterized in that** it consists in passing the biogas through a layer of the product according to any one of Claims 1 to 4.

9. Process according to Claim 8, **characterized in that** the layer is composed of successive and compacted strata with a thickness of 30 to 40 cm.

10. Purification process according to Claim 9, **characterized in that**, for a height of 5 meters of waste, the layer comprises one to three strata of the product, having a height of 40 cm to 1 m.

11. Process according to any one of Claims 8 to 10, **characterized in that** the layer is a final layer, before revegetation, of the waste landfill site.

## Patentansprüche

1. Produkt auf der Basis von Schlacke aus der Verbrennung von Hausmüll und hausmüllähnlichen Abfällen, **dadurch gekennzeichnet, dass** es Folgendes umfasst, in Gew.-%:
- 95 bis 99,5% Schlacken mit einer Teilchengröße von kleiner oder gleich 40 mm und einem Feuchtigkeitsgehalt von 10 bis 30%, vorzugsweise 15 bis 20 Gew.-%; und
- 0,5 bis 5% Koks.

2. Produkt gemäß Anspruch 1, **dadurch gekennzeichnet, dass** es Folgendes umfasst:
- 98 bis 99% Schlacken; und
- 1 bis 2% Koks.

3. Produkt gemäß Anspruch 1 oder 2, **dadurch gekennzeichnet, dass** der Koks ein Braunkohlenkoks oder Thermolysekoks ist.

4. Produkt gemäß einem der Ansprüche 1 bis 3, **dadurch gekennzeichnet, dass** die Schlacke eine Schlacke der Kategorie V oder M gemäß der Norm NF X31-210 ist.

5. Terminale Schicht einer Mülldeponie vor der Neubepflanzung, **dadurch gekennzeichnet, dass** sie aus einem Produkt gemäß einem der Ansprüche 1 bis 4 besteht.

6. Schicht gemäß Anspruch 5, **dadurch gekennzeichnet, dass** sie aus aufeinanderfolgenden kompaktierten Teilschichten von 30 bis 40 cm Dicke besteht.

7. Schicht gemäß Anspruch 6, **dadurch gekennzeichnet, dass** sie auf eine Müllhöhe von 5 m eine bis drei Teilschichten umfasst, die eine Höhe von 40 cm bis 1 m ergeben.

8. Verfahren zur Reinigung von Biogas, das durch eine Mülldeponie erzeugt wurde, **dadurch gekennzeichnet, dass** man das Biogas durch eine Schicht des Produkts gemäß einem der Ansprüche 1 bis 4 leitet.

9. Verfahren gemäß Anspruch 8, **dadurch gekennzeichnet, dass** die Schicht aus aufeinanderfolgenden kompaktierten Teilschichten von 30 bis 40 cm Dicke besteht.

10. Reinigungsverfahren gemäß Anspruch 9, **dadurch gekennzeichnet, dass** die Schicht auf eine Müllhöhe von 5 m eine bis drei Teilschichten aus dem Produkt umfasst, die eine Höhe von ungefähr 40 cm bis 1 m ergeben.

11. Verfahren gemäß einem der Ansprüche 8 bis 10, **dadurch gekennzeichnet, dass** die Schicht eine terminale Schicht der Mülldeponie vor der Neubepflanzung ist.
